# EUROPÄISCHE PATENTSCHRIFT

(11) **EP 1 174 090 B1**
(45) Veröffentlichungstag und Bekanntmachung des Hinweises auf die Patenterteilung: **02.04.2008**
(21) Anmeldenummer: 01116327.6
(22) Anmeldetag: 04.05.1995
(51) Int. Cl.: A61B 17/128, A61B 17/122, A61B 17/02

(54) **Klemme zum Einsatz bei endoskopischen Eingriffen**
Clip for use in endoscopic operations
Pince utilisé pour des interventions endoscopiques

(30) Priorität: 04.05.1994 DE 4415521
(43) Veröffentlichungstag der Anmeldung: 23.01.2002
(62) Teilanmeldung aus: 95917265.1
(73) Patentinhaber: Erbengemeinschaft Dr. hc. Karl Storz, vertreten durch Frau Storz-Reling, Sybill, 78532 Tuttlingen (DE)
(72) Erfinder: Storz, Karl, deceased (DE); Cuschieri, Alfred, Dundee DD1 9SY (GB)
(74) Vertreter: Lohr, Georg

(56) Entgegenhaltungen:
- WO-A-92/13490
- DE-A- 3 044 186
- US-A- 3 958 576
- US-A- 4 337 774
- US-A- 4 706 668
- US-A- 4 957 500
- US-A- 5 242 456
- US-A- 5 304 183

## Beschreibung

### Technisches Gebiet

Die Erfindung bezieht sich auf eine klemme zum Einsatz bei endoskopischen Eingriffen am menschlichen oder tierischen Körper.

Endoskopische Eingriffe werden seit einigen Jahren immer häufiger anstelle von "offenen chirurgischen Eingriffen" ausgeführt. Dabei werden durch einen Kanal eines Einsetzinstruments, beispielsweise eines Arbeitstrokars, die eigentlichen Arbeitsinstrumente, wie Endoskope, Zangen, Scheren usw. in den Körper eingesetzt.

### Stand der Technik

Bei einer Reihe von endoskopischen Eingriffen werden sog. Rohrschaftinstrumente, wie Zangen, Scheren, Clipapplikatoren etc. verwendet, die in den Kanal eines Trokars für die Durchführung des jeweiligen Eingriffs eingesetzt werden. Für eine Reihe von Operationen werden Trokare mit einem Kanal verwendet, dessen lichte Weite nur ca. 5 mm beträgt. Damit ist es in der Regel nicht möglich, in den Kanal eines Trokars gleichzeitig zwei Instrumente einzusetzen. Dies bedeutet, daß bei der Verwendung eines herkömmlichen endoskopischen Instrumentariums immer dann ein weiterer Zugang geschaffen werden muß, wenn es beispielsweise während des Operationsvorgangs erforderlich ist,periphere Arterien mittels einer Klemme zu verschließen.

Gemäß der US-A-5 304 183 wird als nächstliegender Stand der Technik ein Instrument zum Einsatz bei endoskopischen Eingriffen am menschlichen oder tierischen Körper offenbart, das ein Einsetzinstrument, das in den menschlichen oder tierischen Körper einsetzbar ist, und das einen Kanal aufweist, und ein Arbeitsinstrument aufweist, das durch den Kanal des Einsetzinstruments in den Körper einbringbar ist, und das wenigstens aus einem distal angeordneten Arbeitselement und einem länglichen Einführungs- und Betätigungsteil besteht, der den Kanal durchsetzt und der das Arbeitselement mit dem proximalen Bereich des Instruments verbindet, so dass das Arbeitselement vom proximalen Bereich aus betätigt werden kann, wobei das Arbeitselement und das Einführungs- und Betätigungsteil über einen Verbindungsmechanismus derart verbunden sind, dass das Arbeitselement vom Einführungs- und Betätigungsteil intrakorporal lösbar ist, so dass der Betätigungsteil ohne Arbeitselement aus dem Kanal entnommen werden kann, das Arbeitselement nach der Trennung vom Einführungs- und Betätigungsteil wenigstens in einem Funktionszustand verbleibt, und das Arbeitselement nach dem Wiedereinsetzen des Einführungs- und Betätigungsteils in den Kanal des Einsetzinstruments wieder mit diesem verbunden und anschließend durch den Kanal entnommen werden kann.

Der aus der US-A-5 304 183 bekannte Verbindungsmechanismus zwischen distalem Arbeitselement und dem länglichen Einführungs- und Betätigungsteil ist als eine Zapfen- Loch- Verbindung ausgebildet, die eine sehr genaue Querbewegung bei der Wiederverriegelung der Elemente erfordert. Damit ist die Herstellung der Verbindung erschwert. Zudem besteht die Möglichkeit, dass das Arbeitselement unbeabsichtigt im Körper verloren wird.

Gemäß der DE-A-30 44 186 wird ein Klammerhaltegerät zum Abklemmen von Blutgefäßen offenbart, wobei ein Arbeitselement als eine Klammer mit Alpha-Form ausgebildet ist, die in einem "V" endet. Dabei besteht die Klammer aus hinteren Schenkeln, die von einer abgerundeten Spitze unter elastischer Spannung auseinanderlaufen, sich dann kreuzen und in im wesentlichen geraden Schenkeln enden, die den Greifabschnitt der Klammer bilden.

Der Vorgang des Zusammenspiels zwischen der Klammer und dem Haken erfolgt folgendermaßen:

Die Klammer wird über ihre abgerundete Spitze am Ende (Haken) angehakt. D.h. erstens der Haken greift in die Klammer selbst ein -es liegt also keine explizite Öse vor -und zweitens gibt es nur zwei Stellungen (eine Ausgangsstellung und eine zweite bei Drehung des Hakens um 180° um die Längsachse des Stabes an dem sich der Haken befindet) die Haken und Klammer überhaupt zueinander einnehmen können. Die Verbindung beider Elemente wird damit nur in diesen beiden möglichen Stellungen hergestellt oder gelöst. Wird der Haken um die Längsachse gedreht, so dreht sich die angehakte Klammer mit.
Ein Sichern der Verbindung durch ein gegenseitiges Verdrehen von Haken und Klammer wie bei einer Schlüsselloch- Verbindung ist nicht möglich. Die Klammer wird nur durch ein Rohr sicher am Haken gehalten. Befindet sich der Haken außerhalb des Rohrs 13, so ist die Verbindung Haken zu Klammer nicht gesichert. Diese Art der Verbindung kann dazu führen, dass das Arbeitselement unbeabsichtigt im Körper verloren wird.

### Darstellung der Erfindung

Der Erfindung liegt die Aufgabe zugrunde, eine Klemme zum Einsatz bei endoskopischen Eingriffen am menschlichen oder tierischen Körper zu schaffen, die während eines Eingriffs stationär eine bestimmte Aufgabe ausführt, ohne daß für den Einsatz dieser Klemme ein weiterer Zugang zu dem Hohlraum im menschlichen oder tierischen Körper, in dem der Eingriff ausgeführt wird, vorhanden sein müßte.

Zudem soll der Verbindungsmechanismus zwischen des Klemme und länglichem Einführungs- und Betätigungsteil wie gemäß dem Stand der Technik derart weitergebildet werden, dass eine solche Klemme gemäß der US-A-5 304 183 im intrakorporalen Raum einfach und sicher zu betätigen ist.

Eine erfindungsgemäße Lösung dieser Aufgabe ist im Anspruch 1 angegeben.

Hierdurch ist es möglich, zunächst die Klemme beispielsweise durch den Kanal eines als Einsetzinstruments dienenden Trokars einzusetzen, die Klemme im Körperinneren zu positionieren und anschließend das Einführungs- und Bedienteil vom Arbeitselement zu lösen. Das Einführungs- und Bedienteil kann dann aus dem Kanal des Trokars entnommen werden, so daß nunmehr ein weiteres Arbeitsinstrument, wie beispielsweise eine Zange oder eine Schere oder ein Endoskop eingesetzt werden kann. Nach Abschluß der Arbeiten mit dem weiteren Arbeitsinstrument und nach der Funktionserfüllung der in das Körperinnere eingesetzten Klemme wird diese wieder mit dem Einführungs- und Bedienteil verbunden und aus dem Körperinneren entnommen. Selbstverständlich ist es aber auch möglich, die Klemme nach der erneuten Verbindung mit dem Einführungs- und Bedienteil im Körperinneren neu zu positionieren und somit an einer anderen Stelle weiterzuverwenden.

Dabei soll die Klemme nach der Trennung vom Einführungs- und Betätigungsteil wenigsten in einem Funktionszustand verbleiben. Wenn der distale Teil des Instruments eine Klemme ist, die im nicht betätigten Zustand geschlossen ist, kann die Klemme vor Beginn des eigentlichen Operationsvorgangs eingesetzt und so positioniert werden, daß sie z.B. eine periphere Arterie "abklemmt". In diesem Falle ist es bevorzugt, wenn die Klemme in Art einer Buldoc- Klemme ausgebildet ist, wie sie bei offenen Operationen eingesetzt wird. Nach Beendigung des eigentlichen Operationsvorgangs wird dann die Klemme wieder entnommen.

Der Verbindungsmechanismus, der die Klemme und das Einführungs- und Betätigungsteil miteinander lösbar verbindet, ist folgendermaßen ausgebildet:

Die Klemme ist über eine Haken/Ösen- Verbindung mit einem Einführungs- und Betätigungsteil verbunden. Der Einführungs- und Betätigungsteil kann dabei aus einem zylindrischen Rohr und einem Stab bestehen, der in dem Rohr in Richtung seiner Längsachse verschiebbar ist und an seinem distalen Ende den Haken trägt.

Durch die Ausbildung der Haken/Ösen- Verbindung in Art einer Schlüsselloch-Verbindung wird sichergestellt, daß sich die Verbindung nicht unbeabsichtigt löst.

Weiterhin ist es bevorzugt, wenn der Verbindungsmechanismus zumindest axiale Zug- und Druckkräfte auf die Klemme übertragen kann, da hierdurch die Funktionen der Klemme gesteuert werden können, und insbesondere eine einfache Überführung der Klemme in den jeweils gewünschten Funktionszustand möglich ist.

Durch die Weiterbildung, gemäß der der Verbindungsmechanismus einen Schutz gegen unbeabsichtigtes Lösen der Verbindung aufweist, ist sichergestellt, daß die Klemme nicht unbeabsichtigt im Körper verloren wird.

Bevorzugt ist es ferner, wenn der Einführungs- und Betätigungsteil aus einem stabförmigen Teil, der in den Kanal des Einsetzinstruments einsetzbar ist, und einem proximalen Bedienteil besteht. Hierdurch hat das Instrument den gleichen Grundaufbau wie herkömmliche Instrumente, so daß die Bedienungsperson bei der Bedienung nicht grundsätzlich "umlernen" muß. Der proximale Bedienteil kann in an sich bekannter Weise von dem stabförmigen Teil gelöst werden, wie dies von Instrumenten mit der Bezeichnung "Take apart" der Karl Storz GmbH & Co. bekannt ist. Hierdurch wird nicht nur die Reinigung erleichtert, sondern es ist auch möglich, andere Bedienteile, wie beispielsweise anders geformte Handgriffe anzusetzen, wenn einem Benutzer ein Handgriff nicht "zusagt".

Ferner ist es möglich, die Klemme durch Rückzug in den Einführungsteil zu versteifen. Hierdurch wird der Einsetz- und der Entnahmevorgang vereinfacht und darüber hinaus die Gefahr verringert, daß das Arbeitselement versehentlich verloren wird.

### Kurze Beschreibung der Zeichnung

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird. Es zeigen:

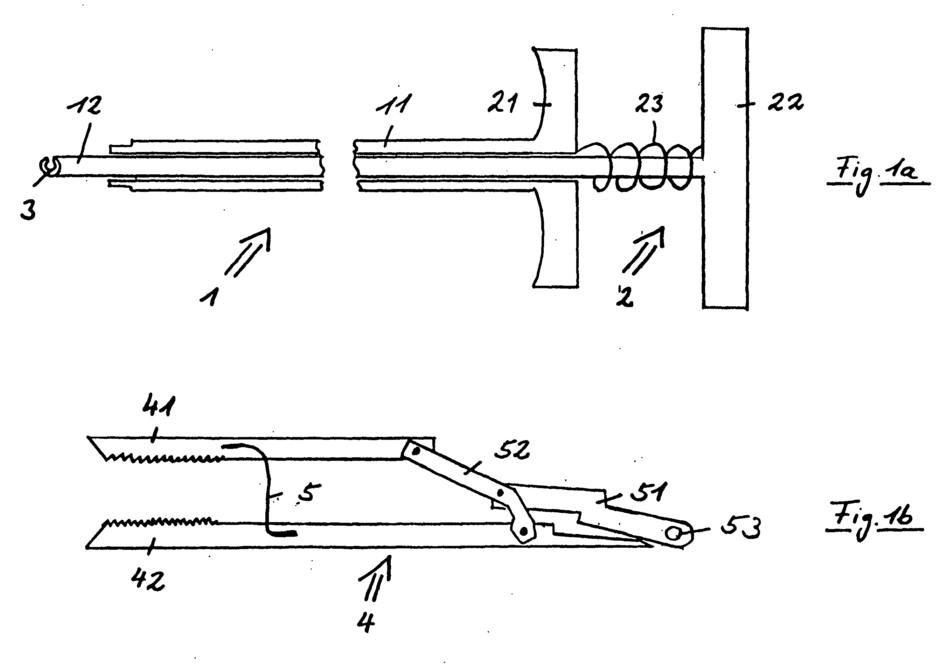

Fig. 1a den Einführungsteil eines ersten Ausführungsbeispiels,
Fig. 1b den distalen Teil des ersten Ausführungsbeispiels,

### Beschreibung von Ausführungsbeispielen

Bei dem in Figur 1 dargestellten Ausführungsbeispiel ist das Arbeitselement des Instruments eine Klemme, die im nicht betätigten Zustand geschlossen ist, und die insbesondere in Art einer Buldoc- Klemme ausgebildet ist.

Der in Fig. 1a dargestellte Einführungs- und Betätigungsteil besteht aus einem stabförmigen Teil I, der in den Kanal eines nicht dargestellten Einsetzinstruments, wie eines Trokars einsetzbar ist, und einem proximalen Bedienteil 2. Der stabförmige Teil 1 wiederum besteht aus einem zylindrischen Rohr 11 und einem Stab 12, der in dem Rohr 11 in Richtung seiner Längsachse verschiebbar ist, und der an seinem distalen Ende einen Haken 3 trägt, der zur Verbindung des Einführungsteils mit dem distalen Teil dient. Der Bedienteil 2 besteht aus zwei Handgriffen 21 und 22, von denen der Handgriff 21 mit dem Rohr 11 und der Handgriff 22 mit dem Stab 12 verbunden ist. Zwischen die Handgriffe 21 und 22 ist eine Feder 23 eingesetzt.

Fig. 1b zeigt die Klemme 4, die den distalen Teil des Instruments bildet, und die in Art einer an sich bekannten Buldoc-Klemme ausgebildet ist. Bei einer derartigen Klemme sind die Maulteile 41 und 42 durch eine Feder 5 so vorgespannt, daß die Maulteile 41 und 42 im nicht betätigten Zustand geschlossen und so vorgespannt sind, daß sie beispielsweise eine Arterie abklemmen können. Durch Verschieben des Stabs 12, dessen Haken 3 in eine Öse 53 an der Klemme eingreift, kann das Maul der Klemme 4 über die Elemente 51 und 52 geöffnet werden. Die Haken/Ösen-Verbindung ist dabei in Art einer Schlüsselloch-Verbindung ausgebildet, so daß die Verbindung nur in einer ganz bestimmten Stellung gelöst werden kann und somit ein unbeabsichtigtes Lösen nicht möglich ist.

Zum Anbringen der Klemme wird das Instrument durch den Kanal eines nicht gezeigten Trokars in den Körper eingesetzt. Nach dem Einsetzen wird das Maul der Klemme 4 geöffnet und beispielsweise eine Arterie abgeklemmt. Anschließend wird die Haken/Ösen-Verbindung gelöst und der Einführungs- und Betätigungsteil entnommen.

Zur Entnahme der Klemme wird genau umgekehrt vorgegangen: Zunächst wird der Haken 3 des Einführungsteils wieder an der Öse 53 angebracht. Durch Verschieben des Stabs 12 wird das Maul der Klemme 4 geöffnet.

Nach der Abnahme der Klemme 4 wird deren Maul wieder geschlossen, zu daß sie durch den Kanal des Trokars entnommen werden kann.

Weiterhin wird bei dem gezeigten Ausführungsbeispiel die Verbindung Klemme/Einführungsteil beim Einsetzen und Entnehmen aus dem Hohlraum durch (teilweisen) Rückzug in das Rohr 11 versteift.

## Patentansprüche

1. Klemme (4) zum Einsatz bei endoskopischen Eingriffen am menschlichen oder tierischen Körper, welche mittels einer Haken/Ösen Verbindung mit einem Einführungs- und Betätigungsteil (1,2) verbunden werden kann, so dass diese Verbindung intrakorporal lösbar und herstellbar ist, und die Klemme (4) nach der Trennung von dem Einführungs- und Betätigungsteil (1,2) in wenigstens einem Funktionszustand verbleibt, wobei
- die Klemme (4) mittels dem Einführungs- und Betätigungsteil (1,2) durch den Kanal eines als Einsetzinstrument dienenden Trokars einsetzbar und entnehmbar ist,
**dadurch gekennzeichnet, dass**
- die Klemme (4) aus zwei Maulteilen besteht, die über die Elemente (51,52) mittels eines Stabes (12) des Einführungs- und Betätigungsteils durch Verschieben geöffnet werden kann,
- die Elemente (51, 52) gelenkig miteinander verbunden sind, und
- die Elemente (51, 52) als Hebel bereitgestellt sind.

2. Klemme nach einem der vorgehenden Ansprüche,
**dadurch gekennzeichnet, dass**
die Klemme im nicht betätigten Zustand geschlossen ist.

3. Klemme nach einem der vorgehenden Ansprüche,
**dadurch gekennzeichnet dass**,
die Haken (3)/ Ösen(53) Verbindung in Art einer Schlüssellochverbindung ausgebildet ist, so dass die Verbindung nur in einer ganz bestimmten Stellung gelöst werden kann, und somit ein unbeabsichtigtes Lösen nicht möglich ist.

4. Klemme nach einem der vorgehenden Ansprüche ,
**dadurch gekennzeichnet, dass**
die Klemme (4) nach der Trennung vom Einführungs- und Betätigungsteil (1,2) zumindest zwei stabile Funktionszustände einnehmen kann.

5. Klemme nach einem der vorgehenden Ansprüche ,
**dadurch gekennzeichnet dass**,
die Haken/Ösen Verbindung zumindest axiale Zug- und Druckkräfte auf die Klemme übertragen kann.

6. Klemme nach einem der vorgehenden Ansprüche,
**dadurch gekennzeichnet dass**,
die Haken/Ösen Verbindung einen Schutz gegen unbeabsichtigtes Lösen der Verbindung aufweist.

7. Klemme nach einem der vorgehenden Ansprüche,
**dadurch gekennzeichnet dass**,
das Einführungs- und Betätigungsteil (1,2) aus einem stabförmigen Teil, der in den Kanal des Einsetzinstruments einsetzbar ist und einem proximalen Bedienteil besteht.

8. Klemme nach Anspruch 8,
**dadurch gekennzeichnet dass**,
der proximale Bedienteil in an sich bekannter Weise von dem Stab gelöst werden kann.

9. Klemme nach einem der vorgehenden Ansprüche,
**dadurch gekennzeichnet dass**,
die Klemme durch Rückzug in den Einführungsteil versteift wird.

10. Klemme nach einem der vorgehenden Ansprüche,
**dadurch gekennzeichnet ,dass**
daß die Klemme (4) mindestens ein Stellelement aufweist, das aus einem Werkstoff mit Formgedächtnis besteht.

11. Klemme nach einem der vorgehenden Ansprüche,
**dadurch gekennzeichnet dass**,
die Klemme in Art einer Bulldoc-Klemme ausgebildet ist.

## Claims

1. A clamp (4) for use during endoscopic surgical interventions on human or animal bodies, which clamp can be connected by means of a hook and eye connection with an insertion and actuation part (1, 2), so that said connection is releasable and producible intracorporally, and the clamp (4) remains in at least one functional state after the separating from the insertion and actuation part (1, 2), with
- the clamp (4) being insertable and removable by means of the insertion and actuating part (1, 2) through the conduit of a trocar which is used as an insertion instrument, **characterized in that**
- the clamp (4) consists of two jaw parts which can be opened by displacement via the elements (51, 52) by means of a rod (12) of the insertion and actuating part,
- the elements (51, 52) are joined to each other in an articulate manner,
- the elements (51, 52) are provided as levers.

2. A clamp according to one of the preceding claims, **characterized in that** the clamp is closed in the non-actuated state.

3. A clamp according to one of the preceding claims, **characterized in that** the hook (3) and eye (53) connection is arranged in the manner of a keyhole connection, so that the connection can be released only in a very specific position and thus inadvertent release is not possible.

4. A clamp according to one of the preceding claims, **characterized in that** the clamp (4) can assume at least two stable functional states after the separation from the insertion and actuation part (1, 2).

5. A clamp according to one of the preceding claims, **characterized in that** the hook and eye connection can transmit at least axial tensile and pressure forces onto the clamp.

6. A clamp according to one of the preceding claims, **characterized in that** the hook and eye connection has a protection against inadvertent release of the connection.

7. A clamp according to one of the preceding claims, **characterized in that** the insertion and actuation part (1, 2) consists of a rod-shaped part which can be inserted into the conduit of the insertion instrument and a proximal operating part.

8. A clamp according to claim 8, **characterized in that** the proximal operating part can be released from the rod in the known manner.

9. A clamp according to one of the preceding claims, **characterized in that** the clamp is stiffened by retraction into the insertion part.

10. A clamp according to one of the preceding claims, **characterized in that** the clamp (4) comprises at least one actuating element which consists of a material with a shape memory.

11. A clamp according to one of the preceding claims, **characterized in that** the clamp is arranged in the manner of a bulldog clamp.

## Revendications

1. Pince (4) destinée à être utilisée dans des opérations endoscopiques sur le corps humain ou animal, qui peut être reliée au moyen d'un assemblage à crochet et oeillet à une partie d'introduction et d'actionnement (1, 2) de telle sorte que cet assemblage puisse être défait et établi et que la pince (4), une fois séparée de la partie d'introduction et d'actionnement (1, 2), reste dans au moins un état fonctionnel,
- la pince (4) pouvant être introduite et retirée au moyen de la partie d'introduction et d'actionnement (1, 2) à travers un canal d'un trocart servant d'instrument d'insertion,
**caractérisée en ce que**
- la pince (4) se compose de deux parties de mors qui peuvent être ouverts par translation à l'aide des éléments (51, 52) au moyen d'une barre (12) de la partie d'introduction et d'actionnement,
- les éléments (51, 52) sont reliés l'un à l'autre de façon articulée, et
- les éléments (51, 52) sont conçus comme des leviers.

2. Pince selon l'une des revendications précédentes, **caractérisée en ce que** la pince est fermée dans l'état non actionné.

3. Pince selon l'une des revendications précédentes, **caractérisée en ce que** l'assemblage par crochet (3) et oeillet (53) est conformé comme un assemblage en trou de serrure, de sorte que l'assemblage ne peut être défait que dans une position très précise et qu'un décrochage accidentel n'est ainsi pas possible.

4. Pince selon la présente invention précédentes, **caractérisée en ce que** la pince (4) peut prendre, après avoir été séparée de la partie d'introduction et d'actionnement (1, 2), au moins deux états fonctionnels stables.

5. Pince selon l'une des revendications précédentes, **caractérisée en ce que** l'assemblage à crochet et oeillet peut transmettre au moins des forces de traction et de compression axiales à la pince.

6. Pince selon l'une des revendications précédentes, **caractérisée en ce que** l'assemblage à crochet et oeillet présente une protection contre un décrochage accidentel de l'assemblage.

7. Pince selon l'une des revendications précédentes, **caractérisée en ce que** la partie d'introduction et d'actionnement (1, 2) se compose d'une partie en forma de barre, qui peut être introduite dans le canal de l'instrument d'insertion, et d'une partie de commande proximale.

8. Pince selon la revendication 8, **caractérisée en ce que** la partie de commande proximale peut être détachée de la barre de manière connue en soi.

9. Pince selon l'une des revendications précédentes, **caractérisée en ce que** la pince est raidie par un retrait dans la partie d'insertion.

10. Pince selon l'une des revendications précédentes, **caractérisée en ce que** la pince (4) présente au moins un élément d'ajustement qui se compose d'un matériau à mémoire de forme.

11. Pince selon l'une des revendications précédentes, **caractérisée en ce que** la pince est conformée comme une pince bulldog.
